# DEMANDE DE BREVET EUROPEEN

(11) **EP 0 970 735 A1**
(43) Date de publication de la demande: **12.01.2000**
(21) Numéro de dépôt: 99390010.9
(22) Date de dépôt: 21.05.1999
(51) Int. Cl.: B01D 19/02, B01D 19/00, C12M 1/04

(54) **Procédé et installation pour le traitement d'un milieu réactionnel susceptible de provoquer un moussage expansif**

(30) Priorité: 11.06.1998 FR 9807365
(71) Demandeur: Biotrade, 31100 Toulouse (FR)
(72) Inventeur: Bermejo, Manuel, 31400 Toulouse (FR); Grasa, Jean-Pierre, 78280 Guyancourt (FR); Pernin, Jean-Marie, 31500 Toulouse (FR)
(74) Mandataire: Cabinet BARRE LAFORGUE & associés

(57) **Abrégé**

L'invention concerne un procédé et une installation pour le traitement d'un milieu réactionnel moussant. Un gaz est introduit au contact de la phase liquide du milieu réactionnel (2) au moyen d'au moins un jet de liquide(s) (16) plongeant dans le milieu réactionnel (2) émis par au moins une buse (15) placée au-dessus de la surface (24) de la phase liquide mais à une hauteur HB inférieure à la hauteur HR des parois latérales (4) de la cuve (1). On limite ainsi la hauteur de mousse formée en évitant tout débordement.

## Description

L'invention concerne le traitement d'un milieu réactionnel, désigné par la suite "milieu réactionnel moussant", comprenant au moins une phase liquide homogène ou hétérogène (comprenant plusieurs liquides non miscibles et/ou au moins une phase solide ou colloïdale telle que des particules en suspension), et une phase gazeuse (un gaz ou un mélange gazeux) introduite au contact de la phase liquide, ce milieu réactionnel étant susceptible, au moins dans certaines conditions, de provoquer un moussage expansif résultant de la formation de bulles de la phase gazeuse dans la phase liquide.

Les milieux réactionnels moussant posent le problème du contrôle du moussage expansif, et ce d'autant qu'il est fréquent que ces phénomènes de moussage expansif apparaissent de façon intempestive, imprévisible, et très rapidement. Or, dans un cadre industriel, le débordement de mousses n'est pas admissible, et ce pour de nombreuses raisons (pollutions, perte de matières, perte de contrôle de la réaction, ...).

Dès lors, ces phénomènes de moussage expansif constituent encore un obstacle majeur à l'exploitation de certains procédés à l'échelle industrielle.

Ainsi, au cours des procédés de fermentation, ou de dégradation biologique aérobie de compositions lipidiques organiques, telles que des déchets graisseux d'origine animale ou végétale, en milieu aqueux, en présence d'une biomasse bactérienne (boue activée), le problème se pose de l'apparition, parfois brutale, de mousses expansives. Ce phénomène est considéré comme un dysfonctionnement limitant notamment le développement des procédés de dégradation biologique aérobie à l'échelle industrielle.

De nombreuses tentatives ont été faites pour pallier ce problème.

Par exemple, FR-2.334.409 préconisait de séparer la mousse produite et de la projeter contre des chicanes d'un brise-mousse. Comme dans les dispositifs cherchant à casser la mousse mécaniquement, cette solution est coûteuse et n'est pas assez efficace en présence d'un moussage expansif brutal et rapide.

FR-2.690.458 décrit un procédé dans lequel on effectue une saponification préalable des graisses, et indique que le développement des mousses doit être stabilisé. Pour ce faire, ce document préconisc d'utiliser une agitation intense provoquée par un débit d'air important, en considérant que le phénomène de moussage expansif serait lié à une agitation insuffisante. Il est néanmoins aussi prévu, à juste titre, d'incorporer un produit anti-mousse au milieu réactionnel.

SU-1.414.793 décrit une installation dans laquelle on récupère la mousse formée par aspiration pour la ré-injecter dans le milieu réactionnel. Cette solution est aussi limitée à des moussages faiblement expansifs.

Le document PATENT ABSTRACT OF JAPAN, Vol. 007, No 056 (C-155) préconisait de détecter la hauteur de mousse formée et, lorsque celle-ci devient trop importante, d'interrompre l'aération du milieu pour supprimer le phénomène de moussage. L'interruption de l'aération a néanmoins comme conséquence d'interrompre la réaction de dégradation, et ne supprime pas la couche de mousse produite en surface. En outre, la quantité d'air présente dans le milieu réactionnel au moment où l'on interrompt l'aération provoque parfois un débordement de la mousse qui continue à se former.

Ainsi, lorsqu'un moussage expansif apparaît, on est obligé, avec les procédés antérieurs, d'interrompre la réaction et donc le fonctionnement normal de l'installation. Or, le moussage expansif pouvant apparaître de façon imprévisible à l'avance, il n'est pas possible de prévoir ces interruptions et donc de les intégrer dans un proccssus industriel dans des conditions satisfaisantes, notamment en termes de productivité. En outre, chaque interruption peut nuire à la réaction. Dans certains cas, il peut être souhaitable de poursuivre la réaction au moins pendant un certain temps après l'apparition du moussage expansif.

L'invention vise donc à pallier ces inconvénients. A ce titre, l'invention vise non pas à empêcher l'apparition des phénomènes de moussage expansif, mais à permettre, lorsqu'un tel moussage expansif se produit, d'empêcher tout débordement de mousse.

Plus particulièrement, l'invention vise à éviter les débordements de mousse sans imposer l'interruption complète du traitement. Ainsi, l'invention vise à permettre, en présence d'un moussage expansif, de continuer le traitement d'un milieu réactionnel moussant en évitant tout débordement de mousse.

L'invention vise aussi à obtenir ce résultat d'une façon extrêmement simple et économique.

Par ailleurs, l'invention vise également à proposer un procédé et une installation de traitement d'un milieu réactionnel moussant -notamment avantageusement applicable pour la fermentation ou pour la dégradation biologique aérobie de compositions lipidiques organiques telles que des déchets graisseux d'origine animale ou végétale en milieu aqueux en présence d'une biomasse bactérienne (boue activée)-, qui soient particulièrement simples, performants, et peu coûteux à l'investissement et à l'exploitation.

A ce titre, l'invention vise à proposer un procédé et une installation de traitement dans lesquels l'introduction de gaz dans le milieu réactionnel et le brassage du milieu réactionnel sont obtenus de façon particulièrement simple -notamment sans nécessiter des dispositifs immergés (chicanes, cheminées, mélangeurs ... )-, et performante -notamment sans laisser de volume mort (non traité) de milieu réactionnel-.

Pour ce faire, l'invention concerne un procédé pour le traitement d'un milieu réactionnel comprenant au moins une phase liquide, et une phase gazeuse introduite au contact de la phase liquide, ce milieu réactionnel étant susceptible de provoquer un moussage expansif, et étant contenu dans une cuve comprenant un fond définissant un niveau inférieur de référence, et des parois latérales s'étendant sur une hauteur HR par rapport au niveau inférieur de référence, caractérisé en ce que :
- on introduit un gaz au contact de la (des) phase(s) liquide(s) du milieu réactionnel au moyen d'au moins un jet de liquide(s) plongeant dans le milieu réactionnel, émis par au moins une buse (une ou plusieurs buses dans le cas de plusieurs jets) placée au-dessus de la surface de la (des) phase(s) liquide(s) du milieu réactionnel, ce jet de liquide(s) plongeant étant adapté pour entraîncr dans le milieu réactionnel le gaz disposé au-dessus du milieu réactionnel, et autour de la buse,
- au moins lorsqu'un moussage expansif apparaît dans la cuve, on interrompt tout éventuel moyen d'introduction de gaz dans le milieu réactionnel autre que le(s) jet(s) de liquide(s) plongeant, et on limite la hauteur de la mousse formée au-dessus du milieu réactionnel en disposant chaque buse à une hauteur HB inférieure à celle HR des parois latérales de la cuve.

En effet, les inventeurs ont constaté que lorsque la mousse arrive à la hauteur de chaque buse émettant un jet de liquide(s) plongeant, en l'absence d'autre source d'introduction de gaz dans le milieu réactionnel, la hauteur de mousse se stabilise et ne dépasse jamais la hauteur de la buse.

Une explication possible de ce phénomène est que la mousse arrivant à la hauteur de la buse, la longueur libre du jet entraînant le gaz perd de son efficacité, de sorte que le gaz n'est alors plus ou quasiment plus entraîné dans le milieu réactionnel.

Il est à noter à cet égard que dans le cadre de l'invention, le(s) jet(s) de liquide(s) plongeant ont pour fonction non seulement d'introduire du gaz dans le milieu réactionnel, mais également de limiter par construction la hauteur de mousse formée résultant de cette introduction de gaz.

Avantageusement et selon l'invention, on utilise exclusivement le(s) jet(s) de liquide(s) plongeant à titre de moyen d'introduction de gaz au contact de la (des) phase(s) liquide(s). Ainsi, il n'est pas nécessairc de surveiller l'apparition du moussage expansif pour interrompre d'éventuels autres moyens d'introduction de gaz dans le milieu.

Dans la majorité des cas, il cst possible de n'utiliser qu'un jet de liquide(s) plongeant unique. Rien n'empêchc ccpendant de prévoir plusieurs jets de liquide(s) plongeant, avec un (des) liquide(s) identique(s) ou distinct(s). Par exemple, avec deux jets de liquide(s) plongeant, il est possible de fonctionner facilement avec une aération réduite de moitié.

Le(s) liquide(s) formant ce(s) jet(s) peut (peuvent) être formé(s) d'au moins une composition liquide de départ alimentant en continu le milieu réactionnel, dans le cas où l'invention est appliquée au traitement d'un milieu réactionnel moussant nécessitant une telle alimentation continue.

En variante, avantageusement et selon l'invention, on extrait en continu du milieu réactionnel, un milieu circulant comprenant au moins une phase liquide que l'on refoule sous pression par au moins une buse formant un jet de liquide(s) plongeant dans le milieu réactionnel.

Avantageusement et selon l'invention, ledit milieu circulant est le milieu réactionnel tel qu'extrait en fond de cuve.

Avantageusemcnt et selon l'invention, chaque buse est disposée fixe, à demeure, à une hauteur HB inférieure à celle HR des parois latérales de la cuve et supérieure à celle HM de la surface de la phase liquide du milieu réactionnel. Rien n'empêche néanmoins de prévoir au contraire des moyens de déplacement d'une buse pour la placer à une telle hauteur HB appropriée uniquement lorsqu'un moussage expansif apparaît.

En outre, avantageusement, un procédé selon l'invention est aussi caractérisé en ce qu'on utilisc une cuve ayant des parois latérales au moins sensiblement cylindriques de révolution, et un fond au moins sensiblement tronconique et en ce qu'on oriente au moins un jet de liquide(s) plongeant avec une inclinaison non nulle par rapport à un plan diamétral de la cuve passant par le point de passage au-dessus des parois latérales de la conduite alimentant la buse, ce jet de liquide(s) plongeant faisant aussi office de moyens de brassage du milieu réactionnel. Avantageusement et selon l'invention, l'inclinaison du jet de liquide(s) plongeant par rapport au plan diamétral est comprise entre 15° et 75°, -notamment est de l'ordre de 30°-. Ainsi, il est à noter que le(s) jet(s) de liquide(s) plongeant a (ont) pour fonctions d'introduire le gaz au sein du milieu réactionnel ; de limiter la formation de mousse à une hauteur prédéterminée et donc d'empêcher tout débordement de mousse ; et d'assurer le brassage du milieu réactionnel.

En outre, avantageusement et selon l'invention, on oriente au moins un jet de liquide(s) plongeant avec une inclinaison non nulle par rapport à la verticale. Avantageusement et selon l'invention, l'inclinaison du jet de liquide(s) plongeant par rapport à la verticale, est comprise entre 10° et 30° -notamment de l'ordre de 20°-.

L'invention s'étend à une installation de mise en oeuvre d'un procédé selon l'invention.

L'invention concerne ainsi une installation pour le traitement d'un milieu réactionnel comprenant au moins une phase liquide, et une phase gazeuse introduite au contact de la phase liquide, et qui est susceptible de provoquer un moussagc expansif, comprenant :
- une cuve destinée à contenir le milieu réactionnel, comportant un fond définissant un niveau inférieur de référence, et des parois latérales s'étendant sur une hauteur HR par rapport au niveau inférieur de référence,
- des moyens d'introduction de gaz au contact de la (des) phase(s) liquide(s) dans le milieu réactionnel,
caractérisée en ce que les moyens d'introduction de gaz consistent en au moins une buse adaptée pour délivrer au moins un jet de liquide(s) plongeant dans le milieu réactionnel adapté pour entraîner dans le milieu réactionnel un gaz disposé au-dessus du milieu réactionnel et autour de la buse, et en ce que la buse est placée :
- au-dessus de la surface de la (des) phase(s) liquide(s) du milieu réactionnel pour que le jet de liquide(s) plonge dans cette (ces) phase(s) liquide(s),
- et à une hauteur HB inférieure à la hauteur HR des parois latérales de la cuve, de sorte que la mousse expansive éventuellement formée ne dépasse pas la hauteur HB de cette busc et ne déborde pas de la cuve.

Avantageusement et selon l'invention, une installation selon l'invention est caractérisée en ce qu'elle est constituéc de la cuve, ct de moyens d'extraction/refoulement en continu pour faire circuler un milieu circulant, comprenant au moins une phase liquide, extrait du milieu réactionnel, ces moyens d'extraction/refoulement comportant :
- au moins une conduite d'extraction de milieu circulant en communication avec le milieu réactionnel contenu dans la cuve,
- des moyens de pompage du milieu circulant aspiré dans chaque conduite d'extraction,
- au moins une conduite de refoulement reliée aux moyens de pompage et comprenant une extrémité dotée d'au moins une buse délivrant au moins un jet de liquide(s) plongeant dans le milieu réactionnel,
lesdits moyens d'extraction/refoulement formant le(s) jet(s) de liquide(s) plongeant ayant simultanément pour fonction :
- d'assurer le brassage du milieu réactionnel,
- d'assurer l'introduction de gaz dans le milieu réactionnel,
- de limiter la hauteur de mousse formée et d'empêcher tout débordement de mousse,
et constituant les seuls moyens de l'installation procurant ces fonctions.

Avantageusement et selon l'invention, le fond de la cuve est de forme au moins sensiblement tronconique. Avantageusement et selon l'invention, la conduite d'extraction est reliée à une extrémité inférieure du fond tronconique de la cuve. Avantageusement et selon l'invention, l'angle au sommet du fond de la cuve est compris entre 30° et 60° -notamment de l'ordre de 45°-.

Par ailleurs, avantageusement et selon l'invention, une installation selon l'invention est caractérisée en ce qu'elle comprend une cuve ayant des parois latérales au moins sensiblement cylindriques de révolution, et un fond au moins sensiblement tronconique, et en ce qu'au moins une buse est orientée avec une inclinaison non nulle par rapport au plan diamétral de la cuve passant par le point de passage au-dessus des parois latérales de la conduite alimentant la buse, le jet de liquide(s) plongeant issu de cette buse faisant office de moyens de brassage du milieu réactionnel. Avantageusement et selon l'invention, l'inclinaison de la buse par rapport au plan diamétral est comprise entre 15° et 75° -notamment est de l'ordre de 30°-.

En outre, avantageusement et selon l'invention, au moins une buse est orientée avec une inclinaison non nulle par rapport à la verticale. Avantageusemcnt et selon l'invention, l'inclinaison de la buse par rapport à la verticale est comprise entre 10° et 30° -notamment de l'ordre de 20°. Avantageusement et selon l'invention, au moins une buse est disposée à une distance de la surface de la (des) phase(s) liquide(s) du milieu réactionnel compris entre le tiers et les deux tiers de la largeur de la cuve au niveau de cette surface.

L'invention concerne aussi un procédé et une installation caractérisés en combinaison par tout ou partie des caractéristiques mentionnées ci-dessus ou ci-après.

L'invention s'étend également à l'application d'un procédé et d'une installation selon l'invention pour réaliser un traitement de dégradation biologique aérobie de compositions lipidiques organiques, telles que des déchets graisseux d'origine animale ou végétale, en milieu aqueux en présence d'une biomasse bactérienne (boue activée) placée dans la cuve. Le milieu réactionnel comprend alors un milieu lipidique aqueux contenant des compositions lipidiques à traiter, un amendement azoté et phosphoré, la biomasse bactérienne, et de l'air et/ou de l'oxygène à titre de gaz introduit dans la phase liquide par le(s) jet(s) de liquide(s) plongeant.

D'autres buts, caractéristiques et avantages de l'invention apparaîtront à la lecture de la description suivante qui se réfère aux dessins dans lesquels :
- la figure 1 est une vue schématique en coupe axiale d'une installation d'un mode de réalisation préférentiel selon l'invention, représentée en l'absence de moussage expansif,
- la figure 2, est une vue schématique similaire à la figure 1 représentant l'installation en présence d'un moussage expansif,
- la figure 3 est une vue schématique de dessus de l'installation de la figure 1,
- la figure 4 est une vue partielle en coupe selon la ligne IV-IV de la figure 3.

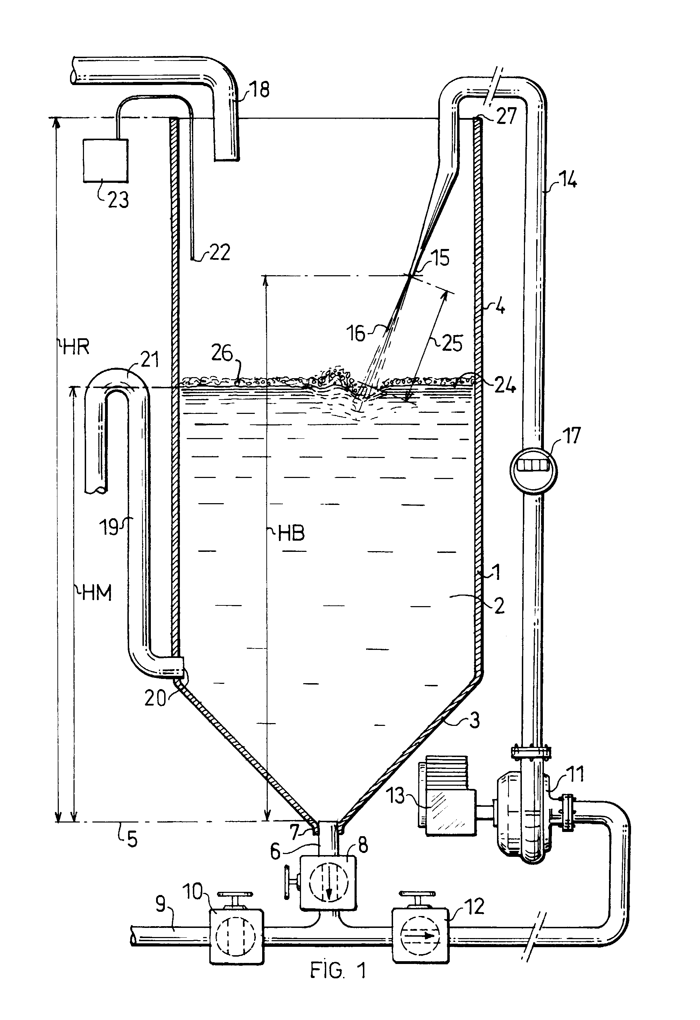

L'installation représentée sur les figures comprend une cuve 1 destinée à contenir un milieu réactionnel 2 comprenant au moins une phase liquide. La cuve 1 comporte un fond 3 inférieur de forme au moins sensiblement tronconique, l'angle au sommet du fond 3 étant compris entre 30° et 60° -notamment de l'ordre de 45° comme représenté figure 1-. La cuve 1 comprend également, au-dessus et à partir du fond 3, des parois latérales 4 qui, dans l'exemple représenté, sont des parois verticales cylindriques de révolution autour de l'axe de symétrie du fond 3 tronconique.

Le fond 3 définit un niveau inférieur de référence 5, par exemple l'extrémité inférieure du fond 3 tronconique. A partir de ce niveau inférieur de référence, on peut définir des hauteurs. Ainsi, les parois latérales 4 s'étendent sur une hauteur HR par rapport au niveau inférieur de référence 5.

Une conduite d'extraction 6 est reliée à l'extrémité inférieure 7 du fond 3 par l'intermédiaire d'une vanne 8 d'extraction. Cette conduite d'extraction 6 est prolongée, d'un côté par une conduite de purge 9 par l'intermédiaire d'une vanne de purge 10. De l'autre côté, la conduite d'extraction 6 est reliée à une pompe 11, par exemple du type centrifuge, par l'intermédiaire d'une vanne 12. La pompe centrifuge 11 est entraînée par un moteur 13 et refoule le milieu extrait par la conduite d'extraction 6 dans une conduite de refoulement 14 qui s'étend vers le haut le long des parois latérales 4 de la cuve, et dont l'extrémité libre est dotée d'une buse 15 disposée fixe à l'intérieur de la cuve 1 pour délivrer un jet de liquide(s) 16 plongeant dans la phase liquide du milieu réactionnel 2. Un débitmètre 17 est interposé sur la conduite de refoulement 14.

Le milieu réactionnel à traiter est introduit dans la cuve 1 par une conduite d'alimentation 18 débouchant au-dessus du niveau du milieu réactionnel 2 contenu dans la cuve 1.

La cuve 1 est dotée de moyens permettant de réguler le niveau du milieu réactionnel 2 dans la cuve 1 à une hauteur HM fixe prédéterminée inférieure à la hauteur HR des parois latérales 4. Pour ce faire, un orifice 20 ménagé en partie inféricure des parois latérales 4 débouche dans une conduite 19 de régulation de niveau qui s'étend à partir de l'orifice 20 vers le haut pour former un coude 21 à la hauteur HM nominale du milieu réactionnel 2. Cette conduite 19 fait office de siphon, de sorte que le niveau du milieu réactionnel 2 ne peut pas dépasser la hauteur HM ainsi définie. Le milieu réactionnel éventuellement évacué par cette conduite 19 de régulation de niveau peut être réintroduit par la conduite d'alimentation 18 si ce milieu réactionnel n'est pas entièrement traité, ou est évacué en tant que milieu traité si le traitement est terminé, une nouvelle quantité de milieu à traiter étant alimentée par la conduite d'alimentation 18.

La cuve 1 est également dotée d'un détecteur de mousse 22 qui peut être constitué d'un fil conducteur électrique dénudé de façon à former un contact électrique lorsque la mousse arrive à la hauteur de ce conducteur électrique. Ce détecteur de mousse 22 est disposé dans la cuve 1 à une hauteur qui est au moins sensiblement la même que celle HB de la buse 15 à l'extrémité libre de la conduite de refoulement 14 ou légèrement supérieure à cette hauteur HB. Le détecteur de mousse 22 est relié à un automate 23 qui gère l'alimentation du milieu réactionnel par la conduite d'alimentation 18.

La hauteur HB de la buse 15 délivrant le jet de liquide(s) 16 plongeant est inférieure à la hauteur HR des parois mais supérieure à la hauteur HM du milieu réactionnel 2 contenu dans la cuve 1. Autrement dit, la buse 15 est placée au-dessus de la surface 24 de la phase liquide du milieu réactionnel 2, de sorte que le jet de liquide(s) 16 plongeant dans cette phase liquide présente une longueur libre 25 apte à entraîner le gaz disposé au-dessus de la phase liquide du milieu réactionnel 2 dans la cuve 1 autour de la buse 15. Ce gaz est, dans l'exemple représenté, l'air atmosphérique, la cuve 1 étant ouverte à sa partie supérieure. Rien n'empêche néanmoins, de prévoir que la cuve 1 soit hermétiquement close à l'aide d'un couvercle, un gaz approprié distinct de l'air atmosphérique pouvant être introduit dans la cuve 1 à sa partie supérieure au-dessus de la phase liquide du milieu réactionnel 2. Ce gaz peut être par exemple de l'oxygène.

Le milieu réactionnel 2 est un milieu réactionnel moussant, c'est-à-dirc qu'une mousse 26 se forme normalement au-dessus de la surface 24 de la phase liquide du milieu réactionnel 2, au cours de la réaction. Dans l'état représenté figure 1, la mousse 26 s'étend sur une faible hauteur au-dessus de la surface 24, aucun phénomène de moussage expansif n'étant constaté. Cette mousse 26 résulte de l'introduction de gaz dans le milieu réactionnel 2 par le jet liquide 16 qui fait également office de brassage du milieu réactionnel 2. En outre, le jet liquide 16 ainsi disposé permet de limiter la hauteur de la mousse 26 expansive éventuellement formée au-dessus de la surface 24 de la phase liquide du milieu réactionnel 2 à la hauteur HB, de sorte que tout débordement de mousse est évité.

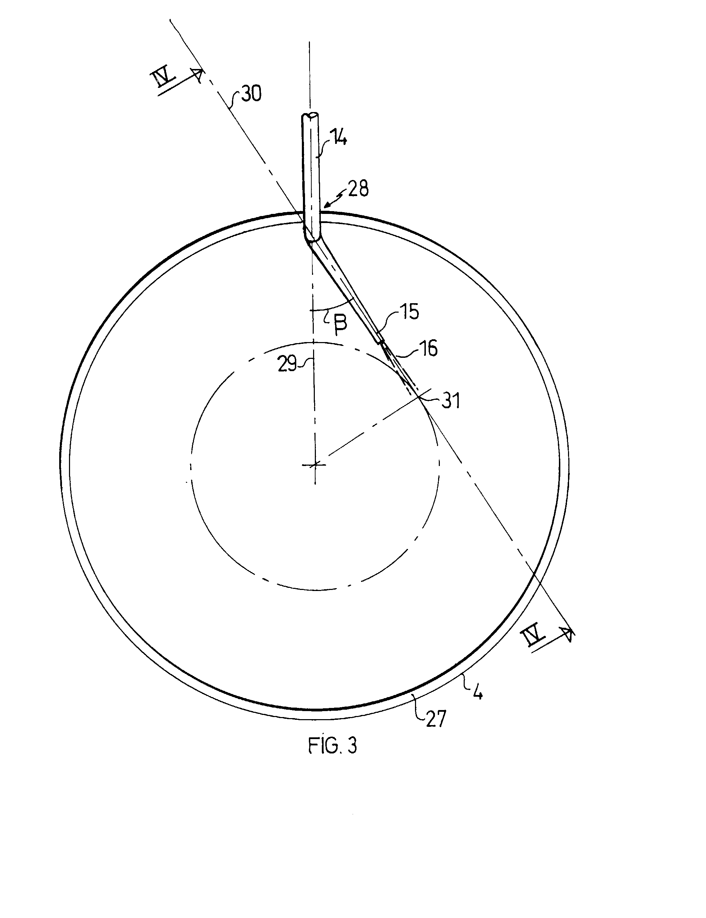

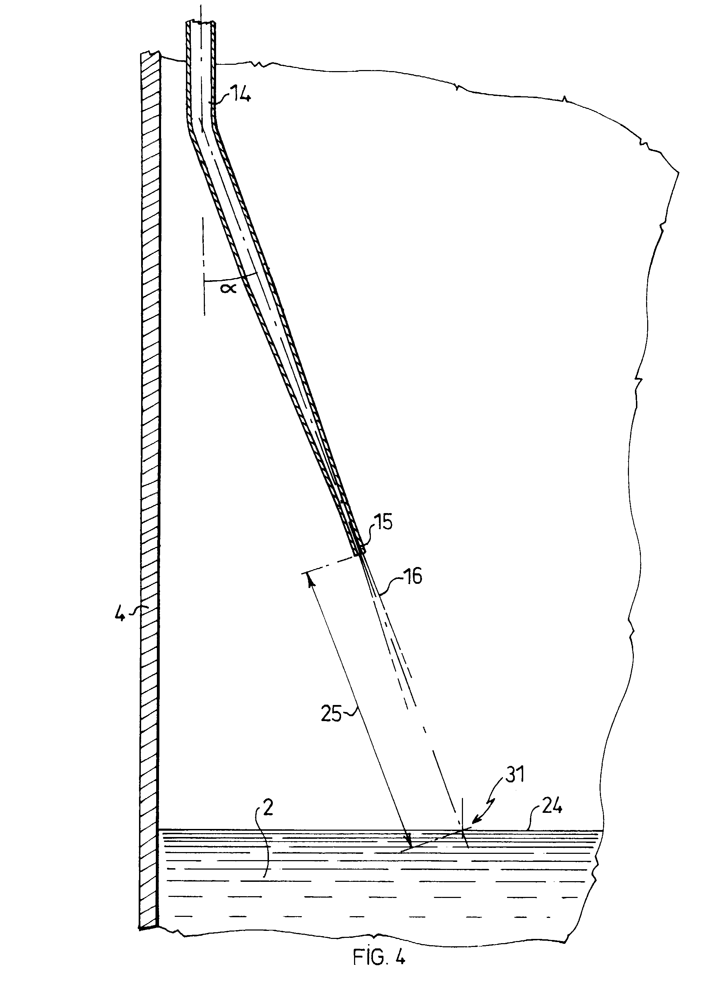

Les figures 3 et 4 représentent plus en détail la forme et l'orientation de la buse 15.

La conduite de refoulement 14 passe au-dessus du bord supérieur libre 27 des parois latérales 4 en un point de passage 28, et est reliée à l'intérieur 1 de la cuve 1 à la buse 15. On peut définir ainsi un plan diamétral 29 vertical de la cuve 1 passant par ce point de passage 28.

La buse 15 s'étend dans un plan vertical 30 qui est incliné d'un angle β par rapport au plan diamétral 29, de sorte que le jet de liquide 16 plongeant dans le milieu réactionnel 2 et qui est issu de la buse 15 fait office de moyen de brassage du milieu réactionnel 2. Il est à noter en particulier que la cuve 1 représentée est exempte de tout autre moyen de brassage ou de mélange, le seul jet de liquide 16 faisant office, avec la forme tronconique du fond 3, de moyen de brassage du milieu réactionnel 2. L'inclinaison β, la forme tronconique du fond 3 et la circulation du milieu réactionnel 2 extrait par la conduite d'extraction 6 et refoulé par la buse 15, assurent un brassage extrêmement efficace du milieu réactionnel 2, ainsi qu'une introduction du gaz dans ce milieu réactionnel 2.

L'inclinaison β peut varier dans une plage de valeurs relativement importantes comprise entre 15° et 75°. Dans l'exemple représenté, l'inclinaison β est de l'ordre de 30° et cette valeur s'est avérée particulièrement efficace et avantageuse. La valeur de l'inclinaison β peut être optimisée en fonction de la nature-notammcnt de la viscosité- du milieu réactionnel 2. Il en va de même de l'inclinaison du fond tronconique 3.

L'angle β peut être orienté pour correspondre à un entraînement du milieu réactionnel 2 par le jet de liquide plongeant 16 dans le sens du vortex naturel, ou en sens contraire au vortex naturel.

En outre, la buse 15 est également orientée avec une inclinaison α par rapport à la verticale et cette inclinaison a est avantageusement comprise entre 10° et 30° -notamment de l'ordre de 20° (figure 3)-.

Les inclinaisons a et β sont déterminées de façon à optimiser l'introduction du gaz dans le milieu réactionnel 2 et le mouvement imparti à la phase liquide du milieu réactionnel 2 par le jet liquide 16 qui arrive à la surface 24 de la phase liquide du milieu réactionnel 2 en un point d'impact 31 situé au moins sensiblement au milieu du rayon de la cuve 1.

Il est également à noter que la buse 15 est disposée à une hauteur de la surface 24 de la phase liquide du milieu réactionnel 2, qui est adaptée notamment selon les performances d'aération recherchées et la géométrie de la cuve 1.

La forme de la buse 15, sa section d'extrémité, la section de la conduite 14 de refoulement qui l'alimente et la pression de refoulement délivrée par la pompe 11 sont également des caractéristiques qui influent sur les caractéristiques du jet liquide 16 et sur son efficacité. Le calcul de ces différents paramètres dimensionnels est bien connu de l'homme de l'art (cf. par exemple "Gaz entrainement by planging liquid jets" Chemical Engeniery Science, Vol. 48, No 21, 1993).

Il est à noter que la buse 15 est orientée pour entraîner le milieu réactionnel 2 en rotation autour de l'axe vertical de la cuve 1. Cette rotation peut être réalisée soit dans le sens du vortex naturel, soit dans le sens contraire du vortex naturel. Cette rotation associée à la forme tronconique du fond 3 permet d'assurer que la totalité du milieu réactionnel 2 est brassée, sans aucun volume mort à l'intérieur de la cuve 1. Ainsi, les particules les plus lourdes contenues dans le milieu réactionnel 2, par exemple la biomasse bactériennc sous forme de flocs dans le cas du traitement de compositions lipidiques par une biomasse bactérienne, sont entraînées vers l'axe et vers l'extrémité 7 inférieure d'extraction du fond 3.

La cuve 1 présente ainsi une structure hydrodynamique de type infiniment mélangée favorable à la mise en oeuvre de nombreuses réactions chimiques ou biochimiques -notamment dans le cadre du traitement biologique des résidus graisseux-.

Il est à noter que la cuve 1 est exempte de toute paroi verticale interne (chicane, cheminée, ...), de tout autre moyen de mélange que le jet liquide 16, et de tout autre moyen d'introduction de gaz que ce jet liquide 16. L'installation selon l'invention est donc particulièrement simple, économique à l'investissement, à l'utilisation, et en terme de maintenance.

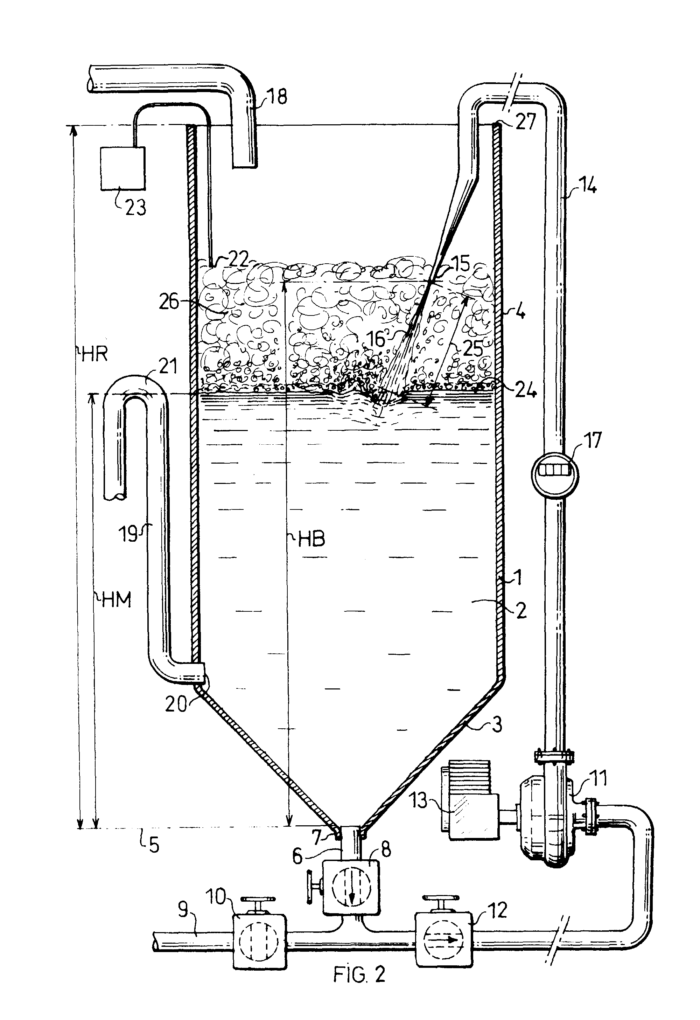

Comme on le voit figure 2, le fait que la buse 15 soit disposée à une hauteur HB inférieure à la hauteur HR des parois latérales 4 permet de limiter la hauteur de la mousse 26 expansive éventuellement formée au-dessus du milieu réactionnel 2. Les inventeurs pensent qu'une explication possible de ce résultat serait que lorsque cette mousse expansive arrive à la hauteur de l'extrémité de la buse 15, le jet liquide 16 est entièrement entouré de mousse et n'entraîne plus ou quasiment plus de gaz supplémentaire apte à former de la mousse à l'intérieur de la phase liquide du milieu réactionnel 2. On constate en tous cas en pratique que la mousse 26 reste cantonnée à une hauteur correspondant au moins sensiblement à la hauteur HB de la buse 15.

En outre, lorsque cette mousse arrive à la hauteur de la buse 15, le phénomène de moussage expansif est détecté par le détecteur de mousse 22. On peut alors casser la mousse expansive ainsi formée par tout moyen approprié, et ce, sans interrompre le jet liquide 16, c'est-à-dire sans interrompre le brassage du milieu réactionnel 2. Dans le cas du traitement de dégradation biologique aérobie de compositions lipidiques, il suffit d'alimenter une quantité appropriée de milieu lipidique aqueux à traiter par la conduite 18 d'alimentation, à une concentration en compositions lipidiques suffisante dans ce milieu, pour constater que le moussage expansif disparaît.

Il est à noter que la hauteur de la mousse expansive formée ne correspond pas en général exactement à la hauteur HB de la buse 15, et dépasse cette hauteur HB d'une valeur qui dépend de la nature de la mousse. Néanmoins, on constate qu'il existe toujours une certaine hauteur de mousse à partir de laquelle le moussage expansif s'arrête, la mousse ne dépassant jamais cette hauteur au-dessus de la buse 15.

Dans le cas du traitement de dégradation biologique aérobie de compositions lipidiques en milieu aqueux en présence d'une biomasse bactérienne, on peut procéder de la façon suivante.

Lorsqu'un moussage expansif est détecté par le détecteur 22, on introduit une quantité de milieu lipidique aqueux à traiter par la conduite 18. Il est à noter que l'on peut maintenir la pompe 11 en fonctionnement de façon à poursuivre la réaction. Si l'on veut interrompre la réaction, on peut aussi au contraire arrêter la pompe. Simultanément, le milieu traité est évacué par la conduite 19 de régulation de niveau. La pompe 11 est alors à nouveau mise en fonctionnement, si elle avait été préalablement arrêtée, et l'on constate que les mousses expansives préalablement formées sont cassées et reviennent à la hauteur nominale de mousse comme représenté figure 1.

Il est à noter que l'ensemble de ce procédé peut être effectué en automatique grâce à l'automate 23.

### Exemple:

Dans cet exemple, la cuve 1 présente un diamètre de 800 mm, un fond tronconique d'angle au sommet de 45° s'étendant sur une hauteur de 370 mm. La surface 24 du liquide définie par la conduite 19 de régulation de niveau est à 1 280 mm du niveau inférieur de référence 5, et les parois latérales présentent une hauteur HR de 1 980 mm. La buse 15 est à 1 630 mm du niveau 5, l'angle a est égal à 20° et l'angle β est égal à 30°. Le volume de travail ainsi défini dans la cuve 1 est de 500 ℓ. La pompe 11 fournit un débit de 3,5 m³/h et la vitesse de sortie du jet liquide 16 est de 8 m/s.

On a traité dans cette installation un milieu lipidique aqueux, le milieu réactionnel comprenant 35 g/ℓ de matière en suspension (biomasse bactérienne). Les compositions lipidiques contenues dans ce milieu lipidique aqueux sont formées de graisses soumises au préalable à une saponification à partir d'une solution de potasse concentrée de façon à former un milieu lipidique aqueux qui est une émulsion aqueuse comprenant une concentration totale en matières extractibles à l'hexane MEHto = 100 g/ℓ. L'automate 23 est programmé pour réaliser une alimentation de 10 ℓ de milieu lipidique aqueux toutes les 8 h, sauf détection d'un moussage expansif par le détecteur 22. Après une première injection de 10 ℓ de milieu lipidique aqueux, définissant le temps initial, la pompe 11 étant mise en fonctionnement, on constate la présence d'une faible hauteur de mousse comme représenté figure 1. Après 6 h de fonctionnement, on détecte l'apparition d'un moussage expansif dans le détecteur 22. On constate que l'on peut maintenir l'installation en fonctionnement sans aucun débordement de mousse. Ainsi, on maintient le fonctionnement pendant 1 h et 50 min supplémentaires et l'on constate que la mousse arrive jusqu'à une hauteur de 100 mm environ au-dessus de la buse 15. On arrête ensuite la pompe 11 pendant environ 10 min puis on alimente à nouveau 10 *ℓ* de milieu lipidique aqueux par la conduite d'alimentation 18. 10 ℓ de milieu traité sont évacués par la conduite 19. Lors du redémarrage de la pompe 11, on constate que la mousse expansive est cassée et revient à la hauteur nominale de faible valeur. On détecte à nouveau ensuite un phénomène de moussage expansif après 5 h 15 min de fonctionnement. On maintient le fonctionnement de la pompe 11 pendant 2 h 35 min supplémentaires et l'on constate que le moussage expansif se maintient, la mousse montant à une hauteur d'environ 100 mm au-dessus de la buse 15 comme précédemment mais ne dépassant pas cette hauteur. On interrompt à nouveau la pompe 1 1 et on alimente une nouvelle fois 10 ℓ de milieu lipidique aqueux.

Les étapes précédentes ont été réitérées un très grand nombre de fois. Aucun débordement de mousse n'a été constaté.

## Revendications

1. Procédé pour le traitement d'un milieu réactionnel (2) comprenant au moins une phase liquide, et une phase gazeuse introduite au contact de la phase liquide, ce milieu réactionnel (2) étant susceptible de provoquer un moussage expansif, et étant contenu dans une cuve (1) comprenant un fond (3) définissant un niveau inférieur (5) de référence, et des parois latérales (4) s'étendant sur une hauteur HR par rapport au niveau inférieur de référence (5), caractérisé en ce que :
- on introduit un gaz au contact de la (des) phase(s) liquide(s) du milieu réactionnel (2) au moyen d'au moins un jet de liquide(s) (16) plongeant dans le milieu réactionnel (2), émis par au moins une buse (15) placée au-dessus de la surface (24) de la (des) phase(s) liquide(s) du milieu réactionnel (2), ce jet de liquide(s) (16) plongeant étant adapté pour entraîner dans le milieu réactionnel (2) le gaz disposé au-dessus du milieu réactionnel (2), et autour de la buse (15),
- au moins lorsqu'un moussage expansif apparaît dans la cuve (1), on interrompt tout éventuel moyen d'introduction de gaz dans le milieu réactionnel autre que le(s) jet(s) de liquide(s) (16) plongeant, et on limite la hauteur de la mousse formée au-dessus du milieu réactionnel (2) en disposant chaque buse (15) à une hauteur HB inférieure à celle HR des parois latérales (4) de la cuve (1).

2. Procédé selon la revendication 1, caractérisé en ce qu'on utilise exclusivement le(s) jet(s) de liquide(s) (16) plongeant à titre de moyen d'introduction de gaz au contact de la (des) phase(s) liquide(s).

3. Procédé selon l'une des revendications 1 à 2, caractérisé en ce qu'on extrait en continu du milieu réactionnel (2) un milieu circulant comprenant au moins une phase liquide que l'on refoule sous pression par au moins une buse (15) formant un jet de liquide(s) (16) plongeant dans le milieu réactionnel (2).

4. Procédé selon la revendication 3, caractérisé en ce que ledit milicu circulant est le milicu réactionnel (2) tel qu'extrait en fond (3) de cuve (1).

5. Procédé selon l'une des revendications 1 à 4, caractérisé en ce qu'on utilise une cuve (1) ayant des parois latérales (4) au moins sensiblement cylindriques de révolution, et un fond (3) au moins sensiblement tronconique et en ce qu'on oriente au moins un jet de liquide(s) (16) plongeant avec une inclinaison (β) non nulle par rapport à un plan diamétral (29) de la cuve (1) passant par le point de passage (28) au-dessus des parois latérales (4) de la conduite (14) alimcntant la buse (15), ce jet de liquide(s) (16) plongeant faisant aussi office de moyens de brassage du milieu réactionnel (2).

6. Procédé selon la revendication 5, caractérisé en ce que l'inclinaison (β) du jet de liquide(s) (16) plongeant par rapport au plan diamétral (29) est comprise entre 15° et 75° -notamment est de l'ordre de 30°-.

7. Procédé selon l'une des revendications 1 à 6, caractérisé en ce qu'on oriente au moins un jet de liquide(s) (16) plongeant avec une inclinaison (α) non nulle par rapport à la verticale.

8. Procédé selon la revendication 7, caractérisé en ce que l'inclinaison (α) du jet de liquide(s) (16) plongeant par rapport à la verticale, est comprise entre 10° et 30° -notamment de l'ordre de 20°-.

9. Installation pour le traitement d'un milieu réactionnel (2) comprenant au moins une phase liquide, et une phase gazeuse introduite au contact de la phase liquide, et qui est susceptible de provoquer un moussage expansif, comprenant :
- une cuve (1) destinée à contenir le milieu réactionnel (2), comportant un fond (3) définissant un niveau inférieur (5) de référence, et des parois latérales (4) s'étendant sur une hauteur HR par rapport au niveau inférieur (5) de référence,
- des moyens d'introduction de gaz au contact de la (des) phase(s) liquide(s) dans le milieu réactionnel (2),
caractérisée en ce que les moyens d'introduction de gaz consistent en au moins une buse (15) adaptée pour délivrer au moins un jet de liquide(s) (16) plongeant dans le milieu réactionnel (2) adapté pour entraîner dans le milieu réactionnel (2) un gaz disposé au-dessus du milieu réactionnel (2) et autour de la buse (15), et en ce que la buse est placée :
- au-dessus de la surface (24) de la (des) phase(s) liquide(s) du milieu réactionnel (2) pour que le jet de liquide(s) (16) plonge dans cette (ces) phase(s) liquide(s),
- et à une hauteur HB inférieure à la hauteur HR des parois latérales (4) de la cuve (2), de sorte que la mousse expansive éventuellement formée ne dépasse pas la hauteur HB de cette buse (15) et ne déborde pas de la cuve (1).

10. Installation selon la revendication 9, caractérisée en ce qu'elle est constituée de la cuve (1), et de moyens (6, 11, 14) d'extraction/refoulement en continu pour faire circuler un milieu circulant, comprenant au moins une phase liquide, extrait du milieu réactionnel, ces moyens (6, 11, 14) d'extraction/refoulement comportant :
- au moins une conduite d'extraction (6) de milieu circulant en communication avec le milieu réactionnel (2) contenu dans la cuve (1),
- des moyens (11) de pompage du milieu circulant aspiré dans chaque conduite d'extraction (6),
- au moins une conduite de refoulement (14) reliée aux moyens (11) de pompage et comprenant une extrémité dotée d'au moins une buse (15) délivrant au moins un jet de liquide(s) (16) plongeant dans le milieu réactionnel (2),
lesdits moyens (6, 11, 14) d'extraction/refoulement formant le(s) jet(s) de liquide(s) (16) plongeant ayant simultanément pour fonction :
- d'assurer le brassage du milieu réactionnel (2),
- d'assurer l'introduction de gaz dans le milieu réactionnel (2),
- de limiter la hauteur de mousse (26) formée et d'empêcher tout débordement de mousse,
et constituant les sculs moyens de l'installation procurant ces fonctions.

11. Installation selon l'une des revendications 9 et 10, caractérisée en ce que le fond (3) de la cuve (1) est de forme au moins sensiblement tronconique.

12. Installation selon les revendications 10 et Il, caractérisée en ce que la conduite d'extraction (6) est reliée à une extrémité inférieure (7) du fond tronconique (3) de la cuve (1).

13. Installation selon l'une des revendications 11 et 12, caractérisée en ce que l'angle au sommet du fond (3) de la cuve (1) est compris entre 30° et 60° -notamment de l'ordre de 45°-.

14. Installation selon l'une des revendications 9 à 13, caractérisée en ce qu'elle comprend une cuve (1) ayant des parois latérales (4) au moins sensiblement cylindriques de révolution et un fond (3) au moins sensiblement tronconique, et en ce qu'au moins une buse (15) est orientée avec une inclinaison (β) non nulle par rapport au plan diamétral (29) de la cuve passant par le point de passage (28) au-dessus des parois latérales (4) de la conduite (14) alimentant la buse (15), le jet de liquide(s) (16) plongeant issu de cette buse (15) faisant office de moyens de brassage du milieu réactionnel (2).

15. Installation selon la revendication 14, caractérisée en ce que l'inclinaison (β) de la buse (15) par rapport au plan diamétral (29) est comprise entre 15° et 75° -notamment est de l'ordre de 30°-.

16. Installation selon l'une des revendications 9 à 15, caractérisée en ce qu'au moins une buse (15) est orientée avec une inclinaison (α) non nulle par rapport à la verticale.

17. Installation selon la revendication 16, caractérisée en ce que l'inclinaison (α) de la buse (15) par rapport à la verticale est comprise entre 10° et 30° -notamment de l'ordre de 20°-.
